# EUROPEAN PATENT APPLICATION

(11) **EP 2 286 760 A1**
(43) Date of publication of application: **23.02.2011**
(21) Application number: 10173126.3
(22) Date of filing: 17.08.2010
(51) Int. Cl.: A61F 2/10, A61M 37/00

(54) **A microneedle roller**

(30) Priority: 17.08.2009 GB 0914273
(71) Applicant: Pangaea Laboratories Limited, 58 Crewys Road London NW2 2AD (GB)
(72) Inventor: Isaacs, Elliot, London, NW2 2AD (GB); Cobbledick, Toby, London, NW2 2AD (GB)
(74) Representative: Hands, Lewis Roger

(57) **Abstract**

Microneedle roller (10,110) comprising a rotatable drum (30,130), the drum including a plurality of radially projecting microneedles (50), wherein the microneedles are provided in substantially linear arrays, each array being carried by a support (125), and each array being arranged in a substantially axial direction relative to the drum.

## Description

The present invention relates to an improved microneedle roller, for use with skin and/or hair and in particular, although not exclusively, as a means to stimulate and/or promote hair growth.

Microneedle rollers may puncture the skin in use and draw blood, although this is not a requirement as mere skin excitation may be all that is required. Accordingly, there is a risk of infection and/or transfer of pathogens.

In a first aspect, the invention provides a microneedle roller comprising a handle and a rotatable drum, the drum including a plurality of radially projecting microneedles, the roller further comprising sterilising means in the form of either or both of an ultrasound generator and a UV light for sterilising one or more of the drum, the microneedles, and the surface over which the drum moves in use.

With these features, the roller is able to provide sterilisation in order to minimise or avoid infection.

The sterilising means may comprise at least the ultrasound generator and further comprise a vibration generating means for imparting a vibration to the roller and/or the surface in use.

The roller may be arranged such that the ultrasound generated by the ultrasound generator and the vibration created by the vibration generating means resonate together. This resonance may provide effective sterilisation but using less electrical power than required otherwise.

The sterilising means may comprise at least the ultrasound generator and further comprise a sterilising fluid.

The handle may comprise a roller hood and/or a comb.

The roller may be arranged such that, in use, an emission from the sterilising means emanates from any one or more of the hood, the handle, the drum and the comb.

The microneedles may be provided in substantially linear arrays, each array being carried by a support, and each array being arranged in a substantially axial direction relative to the drum.

The drum may substantially be a right cylinder which may be hollow, however, oblique rollers are also contemplated, as are other forms, such as a barrel-shape with convex or concave sides, or even a sphere.

The microneedles may be hollow and/or transparent to allow for the direction of fluids, and/or thermal energy, and/or electromagnetic radiation onto or away from a surface in use.

The support may include an abrasion means for abrading the skin. It is known that abrasion may affect the skin, for instance promoting the release of growth-factors. The abrasion means may be a roughened surface or an abrasive material provided adjacent at least one microneedle. The abrasion means may be provided between adjacent microneedles. The abrasion means may be located on the drum surface.

The surface of the drum may provide abrasion by including a suitable outer surface with or without vibration.

The roller may include adjustment means for varying the radial projection of at least some of the microneedles relative to the drum. This is useful as microneedles of different lengths are required for different purposes. For example, relatively long microneedles are required for clinical therapies such as Platelet Rich Plasma, and relatively shorter microneedles are required to improve or provide transdermal drug penetration. Further, a user may wish to use different length microneedles for different treatment types and/or different treatment areas. A means for adjusting the needle length will mitigate the requirement for several fixed length versions.

The radial projection of at least some of the microneedles relative to the outer surface of the drum may be variable between 0 and 5mm. The radial projection of the microneedles relative to the drum may not be variable, and may range between 0.01 and 5mm. Individual microneedles, or microneedle arrays, may differ in radial projection to other microneedles or microneedle arrays on the same roller.

The roller may further comprise treatment means for treating one or more of the drum, the microneedles, the roller and the surface over which the roller moves in use. In this regard the term "treating" is used to mean "affecting" in some way. This may be a chemical, electrical, mechanical, or other type of effect. It may be for the purpose of providing medical, therapeutic and/or cosmetic treatment, and/or for improved operation of the roller.

The treatment means may have sterilisation properties. The sterilisation may occur constantly or intermittently in use. Alternatively, or additionally, the treatment means may have skin and/or hair enhancement properties. Alternatively, or additionally, the treatment means may have pain and/or inflammation controlling properties.

The treatment means may comprise an emission or removal of one or more of a fluid, a liquid, a gas, a sound, a vibration, and an electro-magnetic radiation. It is also possible that the treatment means comprises temperature adjustment means for varying the temperature of the roller and/or the surface in use. The treatment means may comprise a means of irrigating the microneedles and/or the drum and/or a surface in use.

The treatment means may comprise a brush and/or a comb. This may be useful for providing a scrubbing and/or cleaning effect of either the roller and/or the needles and/or the surface in use. Additionally the treatment means may emanate from and/or be conducted or directed by the brush/comb. The brush and/or comb may be useful in controlling the direction of hair as the roller passes across a surface comprising such hairs, preventing the tangling of hair around the roller, thereby facilitating movement of the roller in multiple directions without impedance or degradation of effectiveness.

The treatment means may comprise a light and/or a laser-emitter. The laser may be a cold-beam laser. The light emitted may lie in the ultraviolet or visual ranges although other wavelengths of the light spectrum are contemplated, such as in the range 300 to 900 nm. The treatment means may comprise a plurality of sources of laser and/or non-laser light, of ultraviolet and/or another wavelength.

The treatment means may comprise an ultrasound generator and/or a vibration generating means for imparting a vibration or sound to the roller and/or the surface in use. The ultrasound generator and vibration generating means may also be the sterilising means, described herein.

The fluid may be one or more of a hair-growth stimulant, a fluid containing a growth factor, a sanitising or sterilising fluid, and a pain or inflammation controlling fluid.

The vibration generating means may be one or more of an electrical off-set motor, a piezo-electric crystal and an ultrasound generator. The ultrasound generator may generate ultrasound in a frequency range of 20 kHz to 200 MHz.

The gas emitted may be ozone. The electro-magnetic radiation emitted may be, microwave and/or infra-red. The sound emitted may be ultrasound and/or another frequency sound that may resonate. It may have a frequency lying in the range 20 kHz to 200 MHz.

All of these different physical emissions may be useful in having a beneficial effect on the surface and/or the roller itself. For instance, as at least a partially continuous sterilisation process, and/or a hair growth stimulation process, and/or a pain and/or inflammation controlling process.

The treatment means may emanate from the hood and/or comb. For instance they may act as a light guide for transferring light from a light or laser light source or sources located in the handle and for directing it onto the surface in use, or onto the drum and/or microneedles. Alternatively the source may be located in the hood, comb or brush. The hood, comb or brush may prevent accidental contact with the microneedles by a user.

The vibration and or sound waves may be of an amplitude, wavelength, or resonance, to allow them to be transferred to the roller or surface in use from an emitter in the handle.

In this way, the treatment means may be located in the handle. However, it is also contemplated that the drum comprises the treatment means. It is also possible that the treatment means is located in both or conducted through both or either.

The hood may project substantially radially around the drum. Any or all of the hood, brush and comb may project from the handle, or a supporting arm for the drum. Alternatively any or all of the hood, brush and comb may project radially from one or more arms of the roller, possibly in the form of a circle or part circle, in order to direct the treatment means axially across the drum and/or the surface in use.

The roller may include one or more treatment means and means for a user to choose or select which one or more of these treatment means are in operation at any one time. The roller may include means for disabling any or all of the treatment means as required.

In one embodiment, the microneedles and/or the drum may be electrically connected directly or via the microneedle support(s) to at least a portion of the handle. This may allow, for instance, the charge on the microneedles to be adjusted as desired.

The roller may further include biasing means for resiliently biasing the microneedles radially inward or outward relative to the roller. This biasing means may be a resilient member, such as provided by a thermoplastic elastomer (TPE), provided between the support and a portion of the drum.

The roller may include an integral, and therefore portable, power supply. For instance, the roller may have space for receiving one or more power cells such as batteries. Alternatively, or additionally, the roller may include means for recharging a power cell. For instance, the roller may include means for converting kinetic energy, imparted to the roller in use, or by being shaken, to electrical energy.

The roller may include connection means for connecting the roller to an external power source. The power source may be for supplying power to features of the roller directly, or to recharge an integral power supply.

The roller may comprise materials which are suitable for autoclaving in that the materials do not deteriorate and the performance of the roller is not impaired.

The roller or component parts of the roller may be biodegradable to minimise environmental impact of disposal.

The handle may comprise more than one part, the various parts being removably attachable to one another. For instance, the roller may include a head to which the drum is attached. If it is necessary to replace the drum then it is possible that in one embodiment, the whole of the front part of the roller is removed and a new front part including a new roller is attached to the handle.

The connection between adjacent parts of the roller may include articulation and/or resilient means.

The roller may include torque control means for adjusting the torque required to rotate the drum relative to the handle.

The roller may also be used as part of and/or attached to an assembly that may include a handle extension that may be articulated. The assembly may irrigate the roller or a surface in use with a fluid. The assembly may include gripping means for the invention to be held without using hands, and/or allow multiple rollers to be used at one time. The assembly may include a heating or refrigeration source, a source of electromagnetic radiation, an infrasonic, sonic, or ultrasonic emitter, a comb, brush or other device for styling purposes and/or to part the hair for better microneedle treatment, or any combination of these features.

The roller may be used with a topical treatment including any cosmetically active agent (defined as a compound (natural or synthetic) that has a cosmetic or therapeutic effect on the skin, hair or nails including but not limited to lightening agents, darkening agents, anti-acne agents, shine control agents, anti-microbial agents, anti-inflammatory agents, anti-mycotic agents, anti-parasite agents, external analgesic, sunscreens, photo-protectors, antioxidants, keratolytic agents, detergents or surfactants, moisturisers or humectants, nutrients, vitamins, energy-enhancers, growth factors, anti-perspiration agents, astringents, deodorants, hair-removers, firming agents, anti-callous agents and agents for hair, nail and/or skin conditioning.) Of particular interest are curcumin, taurine, plant sterols, pine bark extract, green tea, red tea, white tea, horsetail extract, marine cartilage, caffeine, kieslerde, copper peptides, copper pca, euk-134, copper(II) 3,5-diisopropylsalicylate, minoxidil and other natural or synthetic nitric oxide donators, finasteride, dutasteride, spironolactone, superoxide dismutase (and mimetics), dimethylmethoxy chromanol, catalase mimetics, saw palmetto and other natural and synthetic anti-dihydrotestosterone agents, hydrolysed lupine protein, vitamins c, a, e, b, f, h, k (and derivatives), bacterial filtrates, glucosamine sulphate, or any combination of these.

The roller may be provided as part of a kit that may comprise a sterilising solution, one or more microneedle roller drums, a handle, a rechargeable power source, a replaceable battery, or a cable for mains electricity with or without a transformer, and a topical treatment as defined above. The kit may be enclosed completely or partially in a case, cover or container for convenient storage and transport of some or all of the articles in the kit.

The roller may be adaptable or modifiable to allow the use of more than one microneedle roller drum at one time, in parallel, tandem, or any other arrangement. This may be achieved by means of expanding the arms of the roller handle, or using a longer axle on an embodiment utilising only one supporting arm. This may additionally or alternatively be achieved by the roller handle being comprised of additional pairs or single supporting arms. In one embodiment, the roller is adapted in this way.

A part or the whole of the roller may be adjustable to change the dimensions of the roller, uses for such a feature may include making it more compact for storage, or increasing the reach of the roller to enable access of different treatment areas. Adjustment may be via a telescopic means, and/or by a means including articulating parts and/or flexible materials. In one embodiment, the roller is adapted in this way.

The roller may be in multiple parts, possibly for the purpose of compact storage, or sterilisation of individual parts.

Any or all of the roller components, and/or the entire roller may be supplied in sterile and/or protective packaging.

The roller may be provided as part of a kit that may comprise any one or more of a sterilising solution, a sanitizing solution, one or more replacement microneedle roller drums which may be fitted to the handle, and one or more arrays of microneedles for fitting to a drum.

The invention may provide a microneedle roller comprising a rotatable drum, the drum including a plurality of radially projecting microneedles, further comprising treatment means for treating one or more of the drum, the microneedles, the roller, and the surface over which the roller moves in use.

Such a microneedle roller may include any one or more features as described in the first aspect.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

Figure 1 is a side view of a roller according to one embodiment of the invention;

Figure 2 is a plan view of the roller of Figure 1;

Figure 3 is a plan view of another embodiment of the invention; and

Figure 4 is a side view of yet another embodiment of the invention;

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn to scale for illustrative purposes. The dimensions and the relative dimensions do not correspond to actual reductions to practice of the invention.

Furthermore, the terms first, second, third and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Similarly, it is to be noticed that the term "connected", used in the description, should not be interpreted as being restricted to direct connections only. Thus, the scope of the expression "a device A connected to a device B" should not be limited to devices or systems wherein an output of device A is directly connected to an input of device B. It means that there exists a path between an output of A and an input of B which may be a path including other devices or means. "Connected" may mean that two or more elements are either in direct physical or electrical contact, or that two or more elements are not in direct contact with each other but yet still co-operate or interact with each other.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may refer to different embodiments. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those skilled in the art. For example, in the following claims, any of the claimed embodiments can be used in any combination.

In the description provided herein, numerous specific details are set forth. However, it is understood that embodiments of the invention may be practised without these specific details. In other instances, well-known methods, structures and techniques have not been shown in detail in order not to obscure an understanding of this description.

The invention will now be described by a detailed description of several embodiments of the invention. It is clear that other embodiments of the invention can be configured according to the knowledge of persons skilled in the art without departing from the true spirit or technical teaching of the invention, the invention being limited only by the terms of the appended claims.

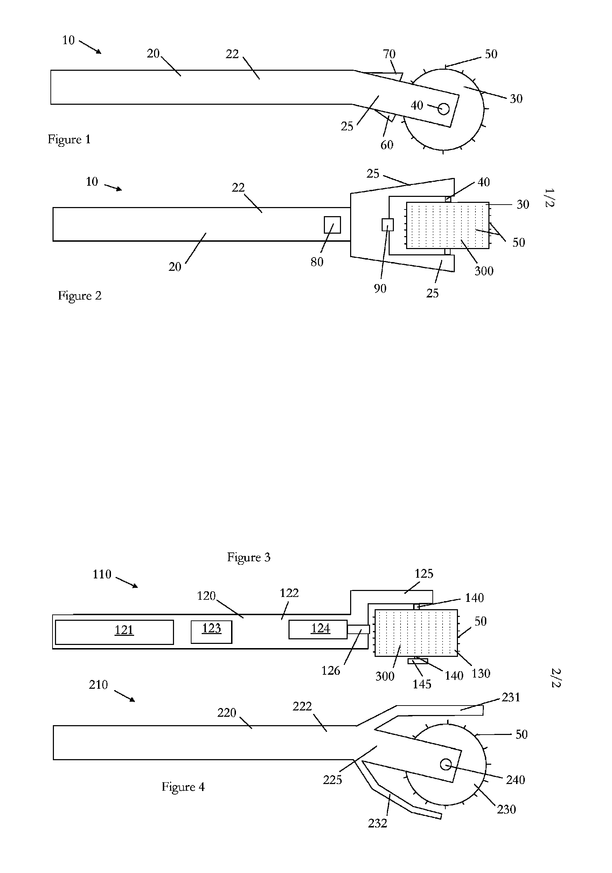

In Figure 1 a microneedle roller 10 comprises a handle 20. The handle 20 itself comprises two parts; a gripping part 20 and a drum support part 25 at one axial end. The two parts may be unitary or detachably connected together.

A drum 30 is provided which supports a plurality of microneedles 50. The drum is supported by the drum support part 25 by means of an axle 40. The axle 40 allows the drum 30 to rotate relative to the handle 20.

In this and all other relevant figures described herein, the number of microneedles is indicative only and should not necessarily be taken as a true representation of the number or arrangement of microneedles of the invention.

The roller 10 also includes emitters 60, 70. These may emit any one or more of a fluid, an electromagnetic radiation and a sound. The emission may be directed either specifically and/or generally in the direction of the roller 30 and/or the microneedles 50 and/or a surface over which the drum 30 is rolled in use.

Figure 2 shows the same roller 10 in plan view. The drum support part 25 is bifurcated such that the drum 30 is supported at each end. The roller 10 also includes another emitter 90 located between the forks of the drum support part 25 such that it may direct emissions towards the roller and/or a surface in use.

The handle 20 also includes a button 80. This button may, when manipulated, move the two forks of the drum support part 25 apart such that the drum 30 is released from the roller 10. Alternatively this button may cause the axle or drum to deform allowing release from the roller.

The other referenced features in Figure 2 are substantially the same as described with regard to Figure 1.

In Figure 3 an alternative roller 110 is shown. It includes a handle 120 comprising a gripping part 122 and a drum support part 125 in a similar manner to that described with reference to Figures 1 and 2.

However, in this embodiment, the drum 130 is only supported at one axial end by the drum support part 125. An axle 140 provides the support between the drum support part 125 and the drum 130. The axle 140 also allows the drum 130 to rotate relative to the handle 120.

The microneedles 50 are present in linear arrays 300 arranged axially across the surface of the drum 130. A button 145 is provided at the opposite axial end of the drum 130 from the drum support part 125. Depression or other manipulation of this button 145 may effect the removal of the drum 130 from the drum support part 125, or adjustment of the radial projection of the microneedles. The handle 120 includes a battery or other power source 121, an ultrasound generator 123 and a light source 124. The light source may emit light via a waveguide 126 onto the drum 130 and/or the microneedles 50 and/or a surface in use. The treatment means 123, 124 although described as an ultrasound generator and a light source may be replaced, or accompanied, by other treatment means such as a fluid source.

Figure 4 shows another embodiment of the invention. This roller 210 includes a handle 220 comprising a gripping part 222 and a drum support part 225 in a similar manner to that described with reference to Figures 1, 2 and 3.

The drum 230 is supported by the drum support part 225. An axle 240 provides the support between the drum support part 225 and the drum 230. The axle 240 also allows the drum 230 to rotate relative to the handle 220.

Furthermore, the roller 210 includes a hood 231 and a comb 232. The hood 231 is positioned substantially radially opposite the comb 232 such that the two partially surround the drum 230 radially. Either, or both, may extend radially around the drum 230 in an arc having a range of 20 degrees to 280 degrees. The hood 231 and/or comb 232 may be integral with, or connected to, the handle 220 and/or the drum support part(s) 225.

Either or both of the hood 231 and the comb 232 may direct treatment means located in the handle 220 towards the drum 230 and/or the microneedles 50 and/or a surface in use.

## Claims

1. A microneedle roller 10, 110, comprising a handle 20, 120 and a rotatable drum 30, 130 the drum including a plurality of radially projecting microneedles 50, the roller further comprising sterilising means in the form of either or both of an ultrasound generator 123 and a UV light 124 for sterilising one or more of the drum, the microneedles, and the surface over which the drum moves in use.

2. The roller of claim 1, wherein the sterilising means comprises at least the ultrasound generator and further comprises a vibration generating means for imparting a vibration to the roller and/or the surface in use.

3. The roller of claim 2, wherein the roller is arranged such that the ultrasound generated by the ultrasound generator and the vibration created by the vibration generating means resonate together.

4. The roller of any preceding claim, wherein the sterilising means comprises at least the ultrasound generator and further comprises a sterilising fluid.

5. The roller of any preceding claim, wherein the handle 20, 120 comprises a roller hood 231 and/or a comb 232.

6. The roller of claim 5, being arranged such that, in use, an emission from the sterilising means emanates from the hood and/or comb.

7. The roller of any preceding claim, being arranged such that, in use, an emission from the sterilising means emanates from the handle and/or the drum.

8. The roller of any preceding claim, further comprising treatment means, wherein the treatment means has skin and/or hair enhancement properties.

9. The roller of claim 8, wherein the treatment means comprises an emission of one or more of a fluid, a sound, a vibration and an electro-magnetic radiation.

10. The roller of either one of claims 8 and 9, wherein the treatment means comprises temperature adjustment means for varying the temperature of the roller and/or the surface in use.

11. The roller of any one of claims 8 to 10, wherein the treatment means comprises a brush and/or a comb.

12. The roller of any one of claims 8 to 11, wherein the treatment means comprises an ultrasound generator and/or a vibration generating means.

13. The roller of claim 12, when dependent on either one of claims 2 and 3, wherein the ultrasound generator and vibration generating means are also the sterilising means.

14. The roller of any one of claims 8 to 13, wherein the treatment means comprises a light and/or a laser-emitter.

15. The roller of claim 9, and/or any one of claims 10 to 14, when dependent either directly or indirectly on claim 9, wherein the fluid is one or more of a hair-growth stimulant, a fluid containing a growth factor, a sanitising or sterilising fluid, and a pain or inflammation controlling fluid.
